# EUROPEAN PATENT APPLICATION

(11) **EP 1 961 726 A1**
(43) Date of publication of application: **27.08.2008**
(21) Application number: 07103009.2
(22) Date of filing: 23.02.2007
(51) Int. Cl.: C07C 43/13, C07C 41/00, C01F 11/02, B01J 23/02

(54) **Process for the conversion of glycerol and catalytically active material suitable therefore**

(71) Applicant: Netherlands Organisation for Scientific Research (Advanced Chemical Technologies For Substantability), 2593 HW Den Haag (NL)
(72) Inventor: Ruppert, Agnieszka M. p/a Universiteit Utrecht,Fac. betawetenschappen, 3584 CA Utrecht (NL); Weckhuysen, Bert M. p/a Universiteit Utrecht,Fac. betawetenschappen, 3584 CA Utrecht (NL)
(74) Representative: van Loon, C.J.J.

(57) **Abstract**

The invention is directed to a process for the conversion of glycerol to produce di-glycerol or higher polyglycerols, which process comprises the steps of converting glycerol at a suitable temperature in the presence of a catalytically active material comprising calcium oxide having a basicity, defined as pK_{BH}+, of between 11 and 15, and recovering di-glycerol and/or higher polyglycerols from the mixture thereby formed.

## Description

The invention is directed to a process for the conversion of glycerol into diglycerol and higher polyglycerols and a catalytically active material suitable therefore.

Glycerol has recently attracted a lot of attention in literature. Being a simple yet highly functionalized molecule, it represents a viable building block for the synthesis of various compounds. The availability of glycerol is also on the rise. More specifically, the production of glycerol as a by-product of the biodiesel manufacturing is increasing, especially in Europe, due to the large fleet of diesel cars, which makes glycerol an attractive and very cheap raw material for the production of bulk as well as fine chemicals. Consequently, new applications for glycerol are currently being actively researched. One of the options is the production of di- and polyglycerols, which are used e.g. in cosmetic and pharmaceutical industries. It is predicted that the market for polyglycerols will significantly develop in the near future.

Conversion of glycerol into higher derivatives thereof, such as diglycerol and polyglycerols, usually occurs through oligomerization in the presence of a basic catalyst. Acrolein can be an undesired byproduct. Generally acrolein is formed by dehydration of glycerol using an acidic catalyst. Thermal decomposition of glycerol can have also a negative impact on the selectivity.

It was found that basic catalysts showed the best catalytic properties in terms of yield and selectivity. Alkaline earth metal oxides showed much better properties in the reaction in comparison with lanthanide oxides. Barium oxide and strontium oxide, having the highest basicity of the alkaline earth metal oxides, are considered to be the most active heterogeneous catalysts, although toxicity arguments could prevent their introduction into a commercial application.

It is an object of the invention to provide a further improved process for the conversion of glycerol into diglycerol or higher polyglycerols, through alkaline catalysis. More in particular it is an object to have an improved process and catalytically active material for this conversion.

The invention is based thereon that, contrary to expectation, when using alkaline earth metal oxides, such as calcium oxide, as the catalytically active material, the best results are not obtained with the highest possible basicity, but with a value in an intermediate range of between 11 and 15 for the pK_{BH+}. In this range of basicity results can be obtained which are better than with e.g. barium oxide and strontium oxide, until now deemed to be the best basic heterogeneous catalysts for this reaction.

In a first embodiment the invention is directed to a process for the conversion of glycerol to produce di-glycerol or higher polyglycerols, which process comprises the steps of converting glycerol at a suitable temperature in the presence of a catalytically active material comprising calcium oxide having a basicity, defined as pK_{BH+}, of between 11 and 15, and recovering di-glycerol and/or higher polyglycerols from the mixture thereby formed.

In further embodiments, the invention is directed to various forms of calcium oxide, which are suitable for use as a catalyst in the process.

In a first form, the calcium oxide is a bulk material, optionally doped with alkali metal, and having a basicity, as herein defined, of between 11 and 15, and a surface area between 20 and less than 90 m²/g. In a second form the calcium oxide is supported on a suitable support, such as the well known (metal) oxides. In a third form the calcium oxide is in the form of colloidal particles.

The calcium oxide material of the invention can be prepared in various ways. In the most preferred embodiment it is prepared by hydrolysis of calcium alkoxide in an organic solvent with water. A process using this method has been described by Koper et al, Chem. Mater. 1993, 5, 500-505. The calcium oxide produced in this method differs in that the surface area of the material is higher. In the present invention this surface area is not very relevant and anyway lower than the lower limit disclosed in the article. The article only refers to the use of this calcium oxide for the destructive adsorption of chlorinated hydrocarbons.

However, it is also possible to prepare calcium oxide by calcination from water-soluble calcium salts, such as from calcium nitrate. Calcium oxide as bulk material can be used as powder, but also in the form of shaped bodies. Particle sizes preferably range from 50 µm to 5 mm.

In case the calcium oxide is to be used on a support, the above methods can be used, in the presence of a support material. Suitable support materials are the support materials that are conventionally used in supporting catalytically active materials, such as metal oxides, silicium oxide, activated carbon and zeolites. These supports may be in the form of powders, shaped bodies, such as extrudates, fibres, monoliths and the like.

In general, the preparation of the calcium oxide includes a calcination step. The temperature of calcination may range from about 200°C to above 750°C. Very good results have been obtained with calcination at a temperature between 300°C and 500°C in the absence of gas flow.

The colloidal-, or nano-particles to be used in the invention, can be prepared by comminuting existing calcium oxide materials. Suitable particle size ranges of these particles are between 50 nm and 500 nm, as determined by static and dynamic light scattering. These particles show a surprisingly high activity and selectivity in the glycerol conversion.

The calcium oxide material may be doped with suitable dopant materials. Especially suitable dopants are the various alkali metal compounds (Li, Na, K, Cs, Rb), more in particular in the metal oxide form. These dopants are present in small amounts, more in particular between 0.1 and 15 wt.%, calculated on the weight of the calcium oxide.

As indicated above, an important feature of the calcium oxide catalyst is the basicity, as defined herein. The value thereof is between 11 and 15. It has surprisingly been found, that in this specific range the optimal results in terms of activity and selectivity are obtained. Preferred is the range of 13.4 to 15.

In addition to the basicity, the Lewis acidity, defined as the Δ u₈ₐ, plays an important role. In case the Lewis acidity is at least 16, in combination with the required basicity of between 11 and 15, the calcium oxide has optimal properties in terms of activity and selectivity.

As has been indicated, the surface area of the calcium oxide is generally between 20 and 90 m²/g for the bulk material. The actual surface area does not play a large role in the activity, but it is, however, to be noted that the best results are obtained within the range specified above. In case of supported calcium oxide the surface area may be much higher.

In the process of the invention glycerol is converted using the above described calcium oxide catalyst. The process can be done in bulk or in the presence of an organic solvent. It is preferred to perform the reaction in the absence of a solvent. It is preferred to use a slurry phase reaction, but it is also possible to use a fixed bed reactor or a structured reactor.

Glycerol, in liquid form, i.e. above the melting point, is reacted in the presence of the catalyst, preferably at a temperature between 125°C and 270°C. Higher temperatures are not preferred, as glycerol tends to decompose at about 290°C. Lower temperatures have the disadvantage of too high a viscosity.

The conversion of glycerol may result in diglycerol, but it is also possible to produce higher glycerols, i.e. polyglycerols, depending on the intended use.

The reaction time will be determined on the basis of the various parameters influencing the reaction, such as temperature, reactor configuration, required end-product and the like.

After completion of the reaction the catalyst is removed from the reaction mixture (in case of slurry phase reaction) and/or the intended reaction products are recovered from the unconverted glycerol, which may be recycled to the reactor.

The properties of the various oxides, i.e. Lewis acidity, basicity and surface area are determined using the following methods.

Lewis acidity was determined by infrared spectroscopy (IR) making use of pyridine as probe molecule. All IR spectra were measured using Perkin Elmer FT-IR 2000 spectrometer. The catalyst materials were pressed into self supporting wafers and activated at 450°C for 1 h under vacuum (0.075 Torr). A quartz cell was used at room temperature for the IR measurements. IR studies of the v(C=C) ring vibrations, especially in the 1400-1700 cm⁻¹ region, is considered as a general method to determine the strength of Lewis acid sites of solid catalyst materials. Pyridine (Acros, 99 %+) adsorption, followed by evacuation, was carried out at room temperature, at a pressure of 11.2 Torr for 1 min. Desorption of pyridine was performed at room temperature at a pressure of 0.075 Torr for 1 h. Examination of the so-called u₈ₐ vibration of adsorbed pyridine, which is located at around 1600 cm⁻¹, is a measure of the Lewis acidity of the catalytic solid and an increasing blue shift from the u₈ₐ of liquid pyridine (1600 cm⁻¹) points towards an increasing Lewis acidity (further denoted as Δ u₈ₐ). The shift, defined as Δ u₈ₐ, is the measure for the Lewis acidity as used herein.

Basicity was determined making use of Hammett indicators. The colour change of the indicators by the interaction with the catalyst materials has been determined making use of UV-Vis-NIR Cary 500 Varian spectroscopy in the diffuse reflectance mode. For this purpose, neutral red (Acros, certified), phenolphthalein (Aldrich, ethanol/water 50%), nile blue (Acros, pure), tropaeolin-o (Acros, pure), clayton yellow (Acros, certified), 2,4-dinitroaniline (Acros, 99 %), 4-chloro-2-nitroaniline (Aldrich, 99 %) and p-dinitroaniline (Acros, certified) have been used. Their pK_{BH+} values are summarized in Table 1.

**Table 1. Hammett indicators.**

| Hammett indicators | pK_{BH+} values of indicators |
|---|---|
| neutral red | 6.8 |
| Phenolphthalein | 8.2 |
| nile blue | 10.1 |
| tropaeolin-O | 11.0 |
| clayton yellow | 13.4 |
| 2,4-dinitroaniline | 15.0 |
| 4-chloro-2-nitroaniline | 17.2 |
| p-dinitroaniline | 18.0 |

In a first step, the catalyst materials were dried at 120°C in air, then 0.25 mg of the sample was shaken with 2 cm³ of a methanol (Biosolve, 99.9 %) solution of the indicator. A change of the colour was observed in between the deprotonated and the neutral form of the indicator. The base strength of the catalyst material, further denoted as pK_{BH+}, is defined between the weakest indicator, which changes the colour and the strongest indicator that gives no colour change. In the case of the colloidal CaO particles the procedure with Hammett indicators was altered. For this purpose, a microscopic glass plate was covered with the glycerol solution (~ 2 ml) containing the colloidal particles and was placed in a Schlenk tube in the oven under vacuum (10⁻³ Torr). To evaporate glycerol, the oven temperature was slowly increased (2°C/min) to 210°C and maintained for 36 h. The film of the remaining colloidal particles, which formed on the plate was analyzed by the UV-Vis microscope (Olympus, LM Plan FL 50X/0,50). A drop of the indicator solution was placed on the plate and after 1 min UV-Vis spectra were collected. The indicator colour change was confirmed by the observed absorption band; i.e., a band at 520 nm for the deprotonated form of Clayton Yellow.

The BET surface areas of the catalyst materials were determined by N₂ sorption measurements at liquid N₂ temperature with a Micromeritics Tristar 3000 apparatus. The samples were outgassed in vacuum at 350°C. The invention is now elucidated on the basis of the following, non limiting examples.

### • Example 1 - Preparation Method A

Different alkaline earth metal oxides, further denoted as MgO-A, CaO-A, SrO-A and BaO-A, have been prepared by performing a high temperature treatment of the corresponding metal nitrates in air in a porcelain crucible in a static manner. In the case of CaO-A, 10 g of Ca(NO₃)₂.4H₂O (Sigma-Aldrich, 99 %) was heated to 700°C for 2 h with a heating rate of 10°C/min. The same procedure has been applied for preparing the MgO-A, SrO-A and BaO-A samples starting from Mg(NO₃)₂ · 4H₂O (Sigma-Aldrich, 99 %), Sr(NO₃)₂ · 4H₂O (Sigma-Aldrich, 99 %) and Ba(NO₃)₂ · 4H₂O (Sigma-Aldrich, 99 %).

### • Example 2 - Preparation Method B

A CaO-B sample was made starting from commercial CaO (J.T. Backer, 96 %) by heating the solid in the presence of distilled water at 80°C for 2 h under continuous stirring, followed by a drying step in a furnace at 120°C in a static manner overnight. The material was then activated under dynamic vacuum (10⁻³ Torr) at different temperatures: In a first step the material was heated to 350°C with a heating rate of 0.5°C/min, followed by maintaining the temperature at 350°C for 1 h; and in a second step, further heating the material to 400°C at a heating rate of 1°C/min and maintaining the temperature at 400°C for 10 h. This treatment was done in a Schlenk tube, which was placed in a furnace.

### • Example 3 - Preparation Method C

The CaO-C sample was prepared from Ca(OH)₂ in the following manner. First, a solution of calcium methoxide was prepared by dissolving 16.8 g metallic Ca (Acros, 99%) in 800 ml dry methanol (Biosolve, 99.9%). The mixture was stirred for 16 h under a flow of Ar to protect calcium methoxide from reacting with atmospheric moisture. This process results in the following reaction:

Ca + 2CH₃OH → Ca(OCH₃)₂ + H₂

Then 150 ml of the Ca(OCH₃)₂ solution was stirred in a beaker with 450 ml of toluene (Acros, 99.99 %). The calcium methoxide material in this mixture was hydrolyzed by the dropwise addition of 8 ml of de-ionized water at room temperature. This process results in the following reaction:

Ca(OCH₃)₂ + 2H₂O → Ca(OH)₂ + 2CH₃OH

The reaction mixture was then transferred into an autoclave and flushed with Ar for 10 min. The autoclave was loaded with Ar to a pressure of 12 bar and heated to 245°C with a temperature gradient of 10°C/min. The final pressure in the autoclave was 39 bar and the temperature of 245 °C was maintained for 15 min. After synthesis, the autoclave was vented, flushed with Ar for 10 min to remove the remaining organic solvents and allowed to cool down to room temperature.

In a final step the obtained calcium hydroxide material was thermally converted to CaO by performing an activation treatment under dynamic vacuum (10⁻³ Torr). For this purpose, the Ca(OH)₂ was placed in a Schlenk tube, outgassed for 20 min and heated according to the following treatment: 25-350°C at a heating rate of 0.5°C/min and maintaining the temperature of 350°C for 1 h; followed by a further heat treatment to 400°C with a heating rate of 1°C/min and maintaining the temperature at 400°C for 1 h.

### • Example 4 - Preparation Method D

A promoted CaO material was prepared by doping CaO (J.T. Backer, 96 %) with 1 wt% K by a wet impregnation method. This sample is further denoted as CaO-D. For this purpose, 10 g of CaO was impregnated with 50 ml of an aqueous solution of KNO₃ (Acros, 99,99 %). The slurry was stirred for 2 h, evaporated to dryness and dried at 120°C for 24 h.

### • Example 5 - Preparation Method E

A CaO-E sample was prepared by stirring 1 g of CaO (J.T. Baker, 99%) in a glycerol (Acros, 99.9%) solution at 220°C under reflux and under a dynamic Ar atmosphere. After 2 h of stirring the remaining CaO was removed by centrifugation of the hot glycerol solution for 20 min (4000 rpm). The top part of the remaining solution was used for further characterization and testing. Light dynamic scattering experiments confirmed the presence of colloidal particles with an average radius of about 150 nm.

The physicochemical properties of the materials prepared in accordance with the above methods, were determined using the methods described above.

### Evaluation of catalyst materials differing in their surface area and acid-base properties

Glycerol etherification was carried out in a stirred batch reactor. 50 g of glycerol (Acros, 99+%) and 1 g of catalyst were stirred at 220°C for at least 20 h under dynamic Ar flow in a three-necked flask of 250 ml equipped with a mechanical stirrer (500 rpm) and a Dean-Stark apparatus with a reflux condenser. Acrolein was collected in the trap with dry ice. Catalysts were fractionated before the reaction and the fraction between 850 and 500 mesh was used. Liquid samples were taken periodically and analyzed by HPLC (Shimadzu, (LC-20AD) equipped with Pathfinder column (4.6 x 250 mm) and RID (RID-10A) detector and autosampler (SIL-20A)). Water was used as an eluent.

**Table 2. Physicochemical and catalytic properties of catalyst materials prepared according to method A (n.d. = not determined).**

| Catalyst material | Δ u₈ₐ [cm⁻¹] | BET (m²/g) | pK_{BH+} | mol glycerol converted per g catalyst after 20 h of reaction |
|---|---|---|---|---|
| MgO-A^{#} | 22 | ~2 | 8.2 > pK_{BH+} > 6.8 | 0.04 |
| CaO-A^{#} | 14 | ~2 | 13.4 > pK_{BH}+ > 11 | 0.29 |
| SrO-A^{#} | n.d. | ~2 | 17.2 > pK_{BH+} > 15 | 0.41 |
| BaO-A^{#} | n.d. | ~2 | 17.2 ≥ pK_{BH+} > 15 | 0.41 |

| | | | | |
|---|---|---|---|---|
| #: not according to the invention | | | | |

**Table 3. Physicochemical and catalytic properties of the different CaO materials under study (n.d. = not determined; n.a. = not applicable).**

| Catalyst material | Δ u₈ₐ [cm⁻¹] | BET (m²/g) | pK_{BH+} | mol glycerol converted per g catalyst after 20 h of reaction |
|---|---|---|---|---|
| CaO-A | 14 | ~2 | 13.4 > pK_{BH+} > 11 | 0.29 |
| CaO-B | 14 | 76 | 15 > pK_{BH+} > 13.4 | 0.22 |
| CaO-C | 18 | 54 | 15 > pK_{BH+} > 13.4 | 0.52 |
| CaO-D | 16 | 20 | 15 > pK_{BH+} > 13.4 | 0.40 |
| CaO-E | n.d. | n.a. | 15 > pK_{BH+} > 13.4 | 1.45 |

## Claims

1. Process for the conversion of glycerol to produce diglycerol or higher polyglycerols, which process comprises the steps of converting glycerol at a suitable temperature in the presence of a catalytically active material comprising calcium oxide having a basicity, defined as pK_{BH+}, of between 11 and 15, and recovering diglycerol and/or higher polyglycerols from the mixture thereby formed.

2. Process according to claim 1, wherein the said catalytically active material has a surface area of less than 90m²/g, preferably between 20 and 90 m²/g.

3. Process according to claim 1 or 2, wherein the material has a Lewis acidity, defined as Δ u₈ₐ of pyridine, of at least 16.

4. Process according to claim 1-3, wherein the said material consist substantially only of calcium oxide.

5. Process according to claim 1-3, wherein the material is present on a support material

6. Process according to claim 4 or 5, wherein the material consist of calcium oxide, doped with at least one alkali metal compound.

7. Process according to claim 1-6, wherein the conversion is performed in the absence of solvent.

8. Process according to claim 1-7, wherein the temperature is chosen between 125 and 270°C.

9. Catalytically active material suitable for the conversion of glycerol to produce di-glycerol or higher polyglycerols according to claim 1, comprising calcium oxide having a basicity, as herein defined, of between 11 and 15, and a surface area of between 20 and less than 90 m²/g.

10. Catalytically active material suitable for the conversion of glycerol to produce di-glycerol or higher polyglycerols according to claim 1, comprising calcium oxide on a support material, said material having a basicity, as herein defined, of between 11 and 15.

11. Catalytically active material suitable for the conversion of glycerol to produce di-glycerol or higher polyglycerols according to claim 1, comprising calcium oxide in colloidal form, said calcium oxide having a basicity, as herein defined, of between 11 and 15.

12. Material according to claim 9-11, wherein the material has a Lewis acidity, defined as Δ u₈ₐ of pyridine, of at least 16.
